# EUROPEAN PATENT SPECIFICATION

(11) **EP 4 048 243 B1**
(45) Date of publication and mention of the grant of the patent: **21.10.2026**
(21) Application number: 20878459.5
(22) Date of filing: 21.10.2020
(51) Int. Cl.: A61K 9/14, A61K 9/51, A61K 31/05, A61K 31/352, A61K 47/40

(54) **SUPERFINE COMPOUNDS AND PRODUCTION THEREOF**
FEINSTVERBINDUNGEN UND IHRE HERSTELLUNG
COMPOSÉS SUPERFINS ET PRODUCTION ASSOCIÉE

(30) Priority: 21.10.2019 US 201962923726 P; 01.11.2019 US 201962929455 P
(43) Date of publication of application: 31.08.2022
(73) Proprietor: Esolate Ltd, Burlington, ON L7P 3A7 (CA)
(72) Inventor: JACKSON, H. Matthew, Burlington, Ontario L7L 2K2 (CA); OH, Jinhee, Hamilton, Ontario L8L 1N6 (CA); BRYSON, Julian, London W4 1HW (GB)
(74) Representative: Wichmann, Hendrik
(86) International application number: PCT/US2020/056731
(87) International publication number: WO 2021/081140

(56) References cited:
- KR-A- 20160 044 925
- US-A1- 2004 034 108
- US-A1- 2005 153 931
- US-A1- 2005 153 931
- US-A1- 2007 072 939
- US-A1- 2019 038 995
- US-A1- 2019 183 848
- US-A1- 2019 241 536
- IVANOVA G. I. ET AL.: "High-pressure NMR characterization of triacetyl-[beta]-cyclodextrin in supercritical carbon dioxide", vol. 47, no. 2, 14 November 2008 (2008-11-14), GB, pages 133 - 141, XP093149241, ISSN: 0749-1581, Retrieved from the Internet <URL:https://api.wiley.com/onlinelibrary/tdm/v1/articles/10.1002%2Fmrc.2365> DOI: 10.1002/mrc.2365

## Description

### CROSS-REFERENCE TO RELATED APPLICATIONS

The present application claims priority to U.S. Provisional Application No. 62/923,726, filed October 21, 2019, and to U.S. Provisional Application No. 62/929,455, filed November 1, 2019.

### FIELD OF INVENTION

The present application relates to stable pharmaceutical grade highly bioavailable superfine cyclodextrin-encapsulated active pharmaceutical ingredients having 99% purity, and to methods of manufacturing the superfine cyclodextrin-encapsulated active pharmaceutical ingredients.

### BACKGROUND OF INVENTION

Lipophilic active pharmaceutical ingredients (APIs) are poorly soluble in water, and their extraction and refinement are time-consuming processes that require extraction, distillate production, and refinement. These processes involve the use of hazardous solvents and often yield products suffering from low stability and lack of efficacy. In addition, the resulting API products lack pharmaceutical grade purity and have poor bioavailability.

Cannabinoids are lipophilic APIs, which are naturally produced in the annual plants *Cannabis sativa, Cannabis indica, Cannabis ruderalis,* and hybrids thereof. Tetrahydrocannabinol (THC), the most active naturally occurring cannabinoid, is beneficial in the treatment of a wide range of medical conditions, including glaucoma, AIDS wasting, neuropathic pain, treatment of spasticity associated with multiple sclerosis, fibromyalgia, emesis and chemotherapy-induced nausea. Cannabidiol (CBD) has no psychotropic effects and it is FDA-approved for the treatment of epilepsy. Cannabinol (CBN) is an effective sedative and inflammation reliever. There is increasing demand for cannabinoids in general, and THC, CBD and CBN in particular, for recreational use. Psychoactive drugs, such as psychedelics, are also in demand for their effects on consciousness state. Solubility in water of these APIs, however, is limited. The solubility in water of cannabidiol (CBD) isolates currently available, for example, is just 0.0126 mg/ml.

Cannabinoids derive from the precursor cannabigerolic acid (CBGA), or its analog cannabigerovaric acid (CBGVA). Enzymatic conversion of CBGA produces a wide variety of cannabinoids, including (-)-trans-Δ9-tetrahydrocannabinol (Δ9-THC), cannabigerol (CBG), cannabichromene (CBC), cannabicyclol (CBL), cannabidiol (CBD), cannabinodiol (CBND), and cannabinol (CBN). Enzymatic conversion of CBGVA produces Δ9-tetrahydrocannabivarin (Δ9-THCV), cannabivarin (CBV), cannabidivarin (CBDV) and cannabichromevarin (CBCV).

There is a need in the art for efficient and safe production of stable pharmaceutical grade, edible, inhalable, soluble, and drinkable highly bioavailable and pure lipophilic API compounds.
Cyclodextrin complexes comprising a cannabinoid are described in US 2005/0153931 A1. High-pressure NMR studies to obtain information on the molecular structure and dynamics of triacetyl-β-cyclodextrin in supercritical carbon dioxide at 313.15K and 20 MPa and its ability to form inclusion complexes under these conditions is described in Ivanova et al., Magn. Reson. Chem. 2009, 47, 133-141. Peracetylated cyclodextrin and drug inclusion particles prepared using supercritical carbon dioxide are described in KR 10-2016-0044925.

### SUMMARY OF INVENTION

The present application presents solutions to the aforementioned challenges, by providing quick, cost-effective and easily scalable processes that produce stable edible, inhalable, soluble or drinkable highly bioavailable superfine cyclodextrin-encapsulated active pharmaceutical ingredients of pharmaceutical grade purity. The disclosed processes do not require the use of organic solvents and thus satisfy the most restrictive health guideline requirements. The resulting superfine pharmaceutical active ingredients may be used for pulmonary and oral delivery, food and beverage production, and pharmaceutical and medical applications.

Thus, disclosed herein are methods of producing a stable edible, inhalable, soluble or drinkable pharmaceutical grade highly bioavailable fine cyclodextrin-encapsulated active pharmaceutical ingredient having 99% purity, as defined in the claims. These ingredients have a 200% increased bioavailability compared to non- cyclodextrin-encapsulated active pharmaceutical ingredient formulations.

The active pharmaceutical ingredient is a cannabinoid.

The disclosed methods comprise in non-sequential order: (a) dissolving the active pharmaceutical ingredient (API) in supercritical, subcritical, high-pressure gas or liquid carbon dioxide to form an API solution; (b) adding one or more cyclodextrins to the API solution; (c) pumping the carbon dioxide at a set pressure and a set temperature for a pre-determined period of time; (d) depressurizing the API solution; and (e) spraying the API solution, thereby producing a stable edible, inhalable, soluble or drinkable pharmaceutical grade highly bioavailable fine cyclodextrin-encapsulated active pharmaceutical ingredient.

In some embodiments, the produced pharmaceutical grade highly bioavailable fine cyclodextrin-encapsulated active pharmaceutical ingredient is in form of inhalable ultrafine nanoparticles having an average particle size between 100 nm and 40 µm and a size distribution within about 1% and about 50% of the average particle size. The ultrafine nanoparticles are produced by a method that comprises: (i) dissolving the API and one or more acetylated cyclodextrins in supercritical, subcritical, high-pressure gas or liquid carbon dioxide in a reaction chamber; (ii) pumping the carbon dioxide at a set pressure and a set temperature for a pre-determined period of time to obtain an acetylated cyclodextrin-encapsulated API solution; (iii) depressurizing the acetylated cyclodextrin-encapsulated API solution; (iv) spraying the acetylated cyclodextrin-encapsulated API solution into a heated precipitator and through a nozzle to obtain an inhalable ultrafine nanoparticles of acetylated cyclodextrin-encapsulated active pharmaceutical ingredient; and (v) collecting and sorting the inhalable ultrafine nanoparticles of acetylated cyclodextrin-encapsulated active pharmaceutical ingredient by particle size.

In some embodiments, the produced pharmaceutical grade highly bioavailable fine cyclodextrin-encapsulated active pharmaceutical ingredient is in form of inhalable dry powder. The dry powder is produced by a method that comprises: (i) pulverizing hydrophilic cyclodextrin into particles having an average particle size between 100 nm and 5 µm; (ii) dissolving the API and one or more acetylated cyclodextrins in supercritical, subcritical, high-pressure gas or liquid carbon dioxide in a reaction chamber; (iii) pumping the carbon dioxide at a set pressure and a set temperature for a pre-determined period of time to obtain an acetylated cyclodextrin-encapsulated API solution; (iv) depressurizing the acetylated cyclodextrin-encapsulated API solution; (v) adding hydrophilic cyclodextrin particles to the acetylated cyclodextrin-encapsulated API solution to create a hydrophilic cyclodextrin suspension- acetylated cyclodextrin-encapsulated API solution mixture; (vi) spraying the mixture into a heated precipitator and through a nozzle to obtain inhalable ultrafine dry powder of a cyclodextrin-encapsulated active pharmaceutical ingredient; and (vii) collecting and sorting the inhalable ultrafine dry powder of the cyclodextrin-encapsulated active pharmaceutical ingredient by particle size.

In some embodiments, the produced pharmaceutical grade highly bioavailable fine cyclodextrin-encapsulated active pharmaceutical ingredient is in form of a soluble or drinkable solution or suspension. The soluble or drinkable solution or suspension is produced by a method that comprises: (i) dissolving hydrophilic cyclodextrin in a hydrophilic liquid at controlled pressure and temperature to form a hydrophilic cyclodextrin aqueous solution; (ii) dissolving the API in supercritical, subcritical, high-pressure gas or liquid carbon dioxide in a reaction chamber; (iii) pumping the carbon dioxide at a set pressure and a set temperature for a pre-determined period of time to obtain an API solution; (iv) depressurizing the API solution; and (v) spraying the API solution into the hydrophilic cyclodextrin aqueous solution and through a nozzle to obtain a drinkable solution or suspension of a hydrophilic cyclodextrin-encapsulated active pharmaceutical ingredient. Suitable hydrophilic liquids include, but are not limited to, water, juice, syrup, milk and alcoholic or non-alcoholic beverages optionally containing an excipient.

The supercritical, subcritical, high-pressure gas or liquid carbon dioxide may comprise an excipient or dispersing agent. In some embodiments, the disclosed methods may further comprise (vi) converting carbon dioxide into gas; (vii) filtering and pressuring carbon dioxide gas to achieve supercritical, subcritical, high-pressure gas or liquid status; and (viii) recirculating carbon dioxide in the reaction chamber for the next processing.

In some embodiments, the set pressure is in a range between 2,500 psi and 6,500 psi, and the set temperature is in a range between about 40°C and about 50°C.

In some embodiments, depressurization may comprise releasing the API solution through a nozzle for short bursts. The nozzle may have a diameter below 5 µm and the short bursts may be for a time period between 0.1 and 1second.

In some embodiments, the cannabinoid comprises one or more of cannabigerolic acid (CBGA), cannabigerovaric acid (CBGVA, tetrahydrocannabinolic acid (THCA), cannabichromene acid (CBCA), cannabidiolic acid (CBDA), tetrahydrocannabivarinic acid (THCVA), cannabichromevarinic acid (CBCVA), cannabidivarinic acid (CBDVA), (-)-trans-Δ9-tetrahydrocannabinol (Δ9-THC), (-)-trans-Δ9-tetrahydrocannabipherol (Δ9-THCP), cannabigerol (CBG), cannabichromene (CBC), cannabicyclol (CBL), cannabidiol (CBD), cannabinodiol (CBND), cannabinol (CBN), or any mixture thereof. The APIs may be in form of an extract, a distillate, a twice-refined distillate, a triple-refined distillate, or a partially purified isolate prior to processing according to the disclosed methods.

Suitable acetylated cyclodextrins comprise acetylated α-cyclodextrin, acetylated β-cyclodextrin, acetylated γ-cyclodextrin or any mixture thereof. In some embodiments, the API and the one or more acetylated cyclodextrins are in an API: acetylated cyclodextrin molar ratio ranging from 1:0.5 to 1:10. In some embodiments, the API: acetylated cyclodextrin molar ratio is 1:0.5, 1:0.75, 1:1, 1:1.5, 1:2, 1:2.5, 1:3, 1:3.5, 1:4, 1:4.5, 1:5, 1:5.5, 1:6, 1:6.5, 1:7, 1:7.5, 1:8, 1:8.5, 1:9, 1:9.5, or 1:10.

Suitable hydrophilic cyclodextrins include, but are not limited to, hydrophilic α-cyclodextrin, hydrophilic β-cyclodextrin, hydrophilic γ-cyclodextrin or any mixture thereof.

Additionally provided herein are stable edible, inhalable, soluble or drinkable pharmaceutical grade cyclodextrin-encapsulated active pharmaceutical ingredients that are produced by the disclosed methods, as defined in the claims. The stable edible, inhalable, soluble or drinkable pharmaceutical grade cyclodextrin-encapsulated active pharmaceutical ingredients have 99.9% purity and 200% increased bioavailability compared to a non-cyclodextrin-encapsulated active pharmaceutical ingredient formulation, and are highly stable at room temperature for extended periods of time. The active pharmaceutical ingredient is a cannabinoid.

In some embodiments, the pharmaceutical grade cyclodextrin-encapsulated active pharmaceutical ingredient is in form of inhalable nanoparticles having an average particle size between 100 nm and 40 µm and a size distribution within 1% and 50% of the average particle size.

In some embodiments, the pharmaceutical grade cyclodextrin-encapsulated active pharmaceutical ingredient is in form of inhalable ultrafine dry powder having an average particle size between 100 nm and 5 µm.

In some embodiments, the pharmaceutical grade cyclodextrin-encapsulated active pharmaceutical ingredient is in form of a drinkable solution or suspension.

The foregoing and other features of the disclosure will become more apparent from the following detailed description, which proceeds with reference to the accompanying figures.

### BRIEF DESCRIPTION OF THE DRAWINGS

The accompanying drawings, which are incorporated into and constitute a part of this specification, illustrate one or more examples of embodiments and, together with the description of example embodiments, serve to explain the principles and implementations of the embodiments.
**Figure 1A** shows a CBD isolate prior to processing. The CBD isolate has crystalline morphology and a large amount of agglomeration between large particles.
**Figure 1B** shows a CBD distillate after processing at a pressure of 3500 psi and a temperature of 40°C. The resulting distillate particles showed a spherical amorphous morphology and a particle size between 100 nm and 40 µm.
**Figure 2A** shows a 32X magnification of purified CBD nanoparticles complexed with α-cyclodextrin in a cannabinoid: cyclodextrin molar ratio of 1:2.5 w/w (250 mg of CBD complexed with 100 mg α-cyclodextrin), produced by the disclosed methods. The CBD nanoparticles have spherical morphology and a particle size between 100 nm and 40 µm.
**Figure 2B** shows a 200X magnification of purified CBD nanoparticles complexed with α-cyclodextrin in a cannabinoid: cyclodextrin molar ratio of 1:2.5 w/w (250 mg of CBD complexed with 100 mg α-cyclodextrin), produced by the disclosed methods. CBD nanoparticles have spherical morphology and a particle size between 100 nm and 40 µm.
**Figure 3** shows crystals of a CBD isolate prior to processing. The crystals are insoluble in acid and in water.
**Figure 4** shows purified CBD nanoparticles in water after processing. The CBD nanoparticles are completely dissolved in water.
**Figure 5** shows purified CBD nanoparticles in acidic solvent resembling stomach conditions after processing. The CBD nanoparticles are completely dissolved in the acidic solution and the solution is clear.

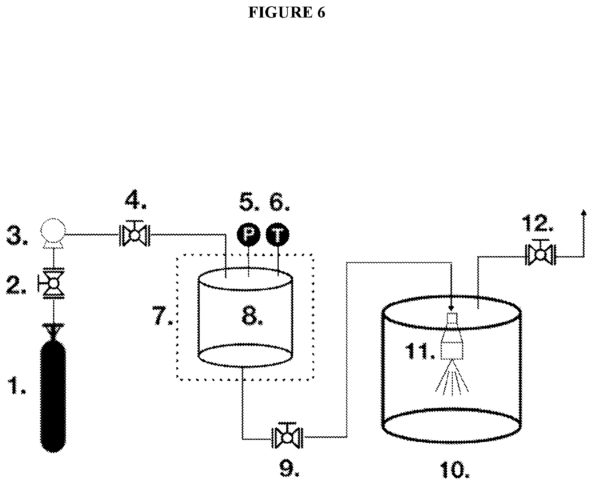

**Figure 6** is a diagram of the equipment used for rapid expansion of supercritical solutions. Canister 1 containing a solvent fluid, such as CO₂ (99.0%); inlet valve 2 opens and controls flow to the inlet for the HPLC pump 3; outlet valve 4 opens and controls the flow of high pressure solvent to the extraction vessel 8; pressure gauge 5 indicates the pressure of the solvent in the inlet line and the extraction vessel 8; temperature gauge 6 indicates the internal temperature of the extraction vessel 8; heating bands 7 regulate the internal heat of the extraction vessel 8; extraction vessel 8 contains the solute to be mixed and dissolved in supercritical fluid; spray valve 9 dispenses the supercritical solution in the extraction vessel through the spray nozzle 11 into the precipitation/collection chamber 10 where the process takes place and the end product is collected; the pressure reaction valve or vent 12 reduces pressure in the precipitation/collection chamber 10.

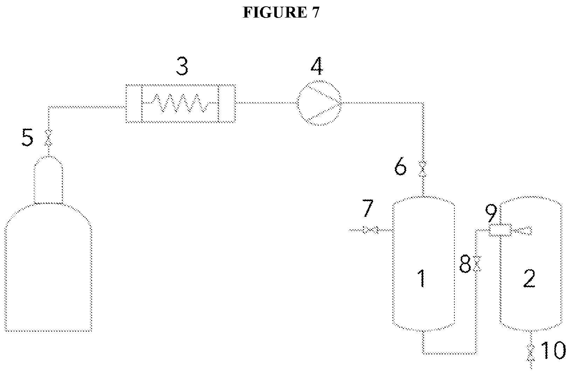

**Figure 7** shows a simplified apparatus for some embodiments of the process provided herein. An API and one or more acetylated cyclodextrins are inserted through a feeding valve into a heated pressurized vessel 1. Supercritical, subcritical, high-pressure gas or liquid carbon dioxide is then released from a CO2 tank through a feeding valve 5, chilled in a cooling chamber 3, and pumped with a pump 4 through an inlet valve 6 into the heated pressurized vessel 1 to dissolve the API and the acetylated cyclodextrins into a cyclodextrin-encapsulated API solution. The solution is then passed through a transfer valve 8, depressurized through a nozzle 9 with short bursts, collected into a powder collection vessel 2, and sorted by particle size through a final product outlet 10.

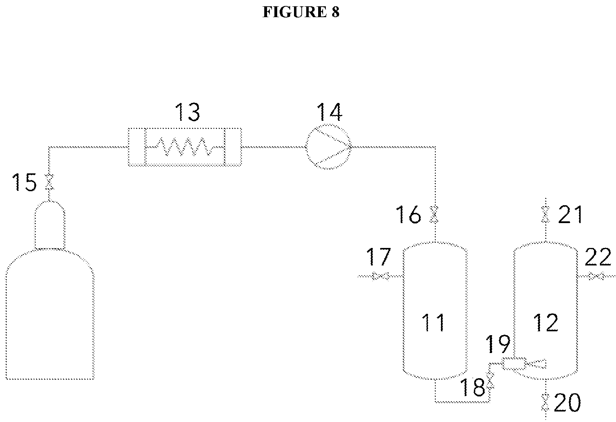

**Figure 8** shows a simplified apparatus for additional embodiments of the process provided herein. One or more hydrophilic cyclodextrins are fed through a feeding valve 22 into a heated pressurized vessel 12 and dissolved in a hydrophilic liquid at a pressure controlled through a pressure control valve 21 and at controlled temperature to form a hydrophilic cyclodextrin aqueous solution. An API is inserted through a feeding valve 17 into a heated pressurized vessel 11. Supercritical, subcritical, high-pressure gas or liquid carbon dioxide is then released from a CO2 tank through a feeding valve 15, chilled in a cooling chamber 13, and pumped with a pump 14 through an inlet valve 16 into the heated pressurized vessel 11 to dissolve the API. The API solution is then passed through a transfer valve 18, and depressurized through a nozzle 19 with short bursts into the heated pressurized vessel 12, where the droplets of API solution are dispersed into the aqueous cyclodextrin solution. The water-soluble hydrophilic API concentrates thus formed are collected through a final product outlet 20.
**Figure 9** shows the dissolution profiles of cyclodextrin-encapsulated API samples as compared to raw API containing equivalent API amounts.

### DETAILED DESCRIPTION OF INVENTION

The following explanations of terms are provided to better describe the present disclosure and to guide those of ordinary skill in the art in the practice of the present disclosure. As used herein, "comprising" means "including" and it is not intended to mean that the compositions and methods exclude elements that are not recited. "Consisting essentially of," when used to define compositions and methods, shall mean excluding other elements of any essential significance to the combination. For example, a composition consisting essentially of the elements as defined herein would not exclude other elements that do not materially affect the basic and novel characteristic(s) of the claimed invention. "Consisting of" shall mean excluding more than a trace amount of other ingredients and substantial method steps recited. The singular forms "a" or "an" or "the" include plural references unless the context clearly dictates otherwise. The term "or" refers to a single element of stated alternative elements or a combination of two or more elements, unless the context clearly indicates otherwise. All numerical designations, e.g., pH, temperature, time, concentration, amounts, and molecular weight, including ranges, are approximations which are varied (+) or (-) by 10%, 1%, or 0.1%, as appropriate. It is also to be understood, although not always explicitly stated, that the reagents described herein are merely exemplary and that equivalents of such are known in the art. Unless explained otherwise, all technical and scientific terms used herein have the same meaning as commonly understood to one of ordinary skill in the art to which this disclosure belongs. The materials, methods, and examples are illustrative only and not intended to be limiting.

To facilitate review of the various embodiments of this disclosure, the following explanations of specific terms are provided:

**Active Pharmaceutical Ingredient:** A biologically active ingredient in a finished product having a direct effect in the diagnosis, cure, mitigation, treatment or prevention of a disease, or in restoring, correcting or modifying one or more physiological functions in a subject, such as a human or animal subject.

**Alcohol:** An organic compound containing a hydroxyl functional group -OH bound to a carbon.

**Analog:** A compound having a structure similar to another, but differing from it, for example, in one or more atoms, functional groups, or substructure. API analogs encompass compounds that are structurally related to naturally occurring APIs, but whose chemical and biological properties may differ from naturally occurring APIs, as well as compounds derived from a naturally occurring API by chemical, biological or a semi-synthetic transformation of the naturally occurring API.

**Cannabinoids:** A class of diverse chemical compounds that activate cannabinoid receptors. Cannabinoids produced by plants are called phytocannabinoids. Typical cannabinoids isolated from the *Cannabis* plants include, but are not limited to, tetrahydrocannabinol (THC), cannabidiol (CBD), cannabigerol (CBG), cannabichromene (CBC), cannabicyclol (CBL), cannabivarin (CBV), tetrahydrocannabivarin (THCV), cannabidivarin (CBDV), cannabichromevarin (CBCV), cannabigerovarin (CBGV), and cannabigerol monomethyl ether (CBGM).

**Cell:** A living biological cell, its progeny or potential progeny, which may be identical or non-identical to the parent cell.

**Contacting:** Placing in direct physical association.

**Co-Solvent:** A solvent added to a fluid in an amount less than 50% of the total volume.

**Cyclodextrins:** A family of cyclic oligosaccharides produced from starch by enzymatic conversion and having a structure comprising a macrocyclic ring of α-D-glucopyranoside units joined by α-1,4 glycoside bonds. Typical cyclodextrins contain six to eight glucose subunits in a ring, creating a cone shape. α-Cyclodextrin contains six glucose subunits; β-cyclodextrin contains seven glucose subunits; and γ-cyclodextrin contains eight glucose subunits. Because cyclodextrins have an inner hydrophobic core and a hydrophilic exterior, they form complexes with hydrophobic compounds.

**Effective amount:** The amount of an active agent (alone or with one or more other active agents) sufficient to induce a desired response, such as to prevent, treat, reduce and/or ameliorate a condition.

**Emulsifier:** A surfactant that reduces the interfacial tension between oil and water, minimizing the surface energy through formation of globules. Emulsifiers include gums, fatty acid conjugates and cationic, anionic and amphotheric surfactants capable of suspending the oily phase and stabilizing the emulsion by coating the oil droplets and avoiding the separation of the internal oily phase. The film coat produced by the emulsifier is a barrier between the immiscible phase and it also prevents droplets association, coagulation and coalescence. Examples of emulsifier include, but are not limited to, lecithin, glyceryl monostearate, methylcellulose, sodium lauryl sulfate, sodium oleate, sorbitan monopalmitate, sorbitan monostearate, sorbitan tristrearate, tragacanth, triethanolamine oleate, polyethylene sorbitan monolaurate, poloxamer, detergents, Tween 80 (polyoxyethylene sorbitan monooleate), Tween 20 (polyoxyethylene sorbitan monolaurate), cetearyl glucoside, polyglucosides, sorbitan monooleate (Span 80), sorbitan monolaurate (Span 20), polyoxyethylene monostearate (Myrj 45), polyoxyethylene vegetable oil (Emulphor), cetyl piridinium chloride, polysaccharides gums, Xanthan gums, Tragacanth, Gum arabica, Acacia, or proteins and conjugated proteins capable of forming and protecting stable oil in glycerin emulsion.

**Hydrophilic:** A polymer, substance or compound that is capable of absorbing more than 10% of water at 100% relative humidity (RH).

**Hydrophobic:** A polymer, substance or compound that is capable of absorbing no more than 1% of water at 100% relative humidity (RH).

**Lipophilic:** A substance or compound that has an affinity for a non-polar environment compared to a polar or aqueous environment.

**Nanoparticle:** A particle of matter measurable on a nanometer scale. Nanoparticles may be in solid or semi-solid form.

**Organic Solvent:** A hydrocarbon-based solvent optionally comprising one or more polar groups capable of dissolving a substance that has low solubility in water.

**Psychedelic Drug:** A hallucinogen that triggers a non-ordinary state of consciousness and psychedelic experiences via serotonin 2A receptor agonism.

**Purification or Purify:** Any technique or method that increases the degree of purity of a substance of interest, such as an enzyme, a protein, or a compound, from a sample comprising the substance of interest. Non-limiting examples of purification methods include silica gel column chromatography, size exclusion chromatography, hydrophobic interaction chromatography, ion exchange chromatography including, but not limited to, cation and anion exchange chromatography, free-flow-electrophoresis, high performance liquid chromatography (HPLC), and differential precipitation.

**Purity:** A quality of an unadulterated, uncontaminated and safe product obtained by the disclosed methods and meeting pharmaceutical standards.

**Recovery:** A process involving isolation and collection of a product from a reaction mixture. Recovery methods may include, but are not limited to, chromatography, such as silica gel chromatography and HPLC, activated charcoal treatment, filtration, distillation, precipitation, drying, chemical derivation, and any combinations thereof.

**Supercritical Fluid:** Any substance at a temperature and pressure above their critical point, where distinct liquid and gas phases do not exist. Solubility of a material in the fluid increases as the density of the fluid increases. Density of the fluid increases with pressure, and at constant density, solubility of a material in the fluid increases as the temperature increases. Exemplary supercritical fluids include, but are not limited to, carbon dioxide, water, methane, propane, ethane, ethylene, propylene, methanol, ethanol, acetone and nitrogen oxide.

**Water-Immiscible:** Any non-aqueous or hydrophobic fluid, liquid or solvent which separates from solution into two distinct phases when mixed with water.

**Water-Insoluble:** A compound or composition having a solubility in water of less than 5%, less than 3%, or less than 1%, measured in water at 20°C.

### Methods of Producing Highly Bioavailable Edible, Inhalable, Soluble or Drinkable Pharmaceutical Grade Pure Active Pharmaceutical Ingredients

The development of efficient processes for the production of pure lipophilic API compounds with high bioavailability has to date been hampered by the low solubility of APIs in aqueous and acidic conditions. As a consequence, classical lipophilic API preparation and refinement is a time-consuming process, which often requires the use of toxic organic solvents. In addition, APIs produced by currently available methods suffer from lack of purity and have low bioavailability.

Disclosed herein are quick and efficient methods that overcome these challenges, by making use of supercritical, subcritical, high-pressure gas or liquid carbon dioxide and acetylated and/or hydrophilic cyclodextrins to create highly pure, ultrafine API-cyclodextrin inclusion complexes that are suitable for pulmonary and oral delivery. The methods provided herein significantly decrease API particle size, do not include the use of toxic organic solvents, and produce pure active pharmaceutical compounds that meet the most restrictive health requirements. Cyclodextrin encapsulation protects the API from degradation after production, and thus the pure active pharmaceutical compounds produced according to the disclosed methods are highly stable for extended periods of time, such as 16 months or longer, at room temperature and do not degrade over time. In addition, since carbon dioxide is a gas at atmospheric pressure, CO₂ removal is much quicker and safer than organic solvent removal, and no residual solvent is left in the final product.

Thus, in some embodiments, a method is provided, that comprises: (i) dissolving the API and one or more acetylated cyclodextrins in supercritical, subcritical, high-pressure gas or liquid carbon dioxide in a reaction chamber; (ii) pumping the carbon dioxide at a set pressure and a set temperature for a pre-determined period of time to obtain an acetylated cyclodextrin-encapsulated API solution; (iii) depressurizing the acetylated cyclodextrin-encapsulated API solution; (iv) spraying the acetylated cyclodextrin-encapsulated API solution into a heated precipitator and through a nozzle to obtain inhalable ultrafine nanoparticles of acetylated cyclodextrin-encapsulated active pharmaceutical ingredient; and (v) collecting and sorting the inhalable ultrafine nanoparticles of acetylated cyclodextrin-encapsulated active pharmaceutical ingredient by particle size.

The disclosed method produces inhalable pharmaceutical grade highly bioavailable ultrafine nanoparticles of cyclodextrin-encapsulated active pharmaceutical ingredients. The inhalable ultrafine nanoparticles have an average particle size between 100 nm and 40 µm and a size distribution within about 1% and about 50% of the average particle size. The superfine nanoparticles may also be added to food products, such as solid foods, beverages, condiments, and nutraceuticals, and may be used for medical and pharmaceutical applications in immediate release, sustained release and controlled release formulation for prolonged and sustainable effects.

In some other embodiments, a method is provided, that comprises: (i) pulverizing hydrophilic cyclodextrin into particles having an average particle size between 100 nm and 5 µm; (ii) dissolving the API and one or more acetylated cyclodextrins in supercritical, subcritical, high-pressure gas or liquid carbon dioxide in the reaction chamber; (iii) pumping the carbon dioxide at a set pressure and a set temperature for a pre-determined period of time to obtain an acetylated cyclodextrin-encapsulated API solution; (iv) depressurizing the acetylated cyclodextrin-encapsulated API solution; (v) adding hydrophilic cyclodextrin particles to the acetylated cyclodextrin-encapsulated API solution to create a hydrophilic cyclodextrin suspension- acetylated cyclodextrin-encapsulated API solution mixture; (vi) spraying the mixture into a heated precipitator and through a nozzle to obtain inhalable ultrafine dry powder of a cyclodextrin-encapsulated active pharmaceutical ingredient; and (vii) collecting and sorting the inhalable ultrafine dry powder of the cyclodextrin-encapsulated active pharmaceutical ingredient by particle size.

The disclosed method produces a pharmaceutical grade highly bioavailable ultrafine inhalable dry powder of cyclodextrin-encapsulated active pharmaceutical ingredients. The particle size of the dry powder may be varied by determining the particle size of the hydrophilic cyclodextrins, which rather than dissolving form a suspension in carbon dioxide. The hydrophobicity of the inhalable dry powder is controlled by adjusting the ratio between acetylated and hydrophilic cyclodextrins. The dry powder thus produced is readily soluble in water, hydrophilic liquids, brewed or fermented alcoholic and non-alcoholic beverages, juices, may be added to food products, such as solid foods, beverages, condiments, and nutraceuticals, and may be used for medical and pharmaceutical applications in immediate release, sustained release and controlled release formulation for prolonged and sustainable effects.

In additional embodiments, a method is provided, that comprises: (i) dissolving hydrophilic cyclodextrin in a hydrophilic liquid at controlled pressure and temperature to form a hydrophilic cyclodextrin aqueous solution; (ii) dissolving the API in supercritical, subcritical, high-pressure gas or liquid carbon dioxide in a reaction chamber; (iii) pumping the carbon dioxide at a set pressure and a set temperature for a pre-determined period of time to obtain an API solution; (iv) depressurizing the API solution; and (v) spraying the API solution into the hydrophilic cyclodextrin aqueous solution and through a nozzle to obtain a drinkable solution or suspension of a hydrophilic cyclodextrin-encapsulated active pharmaceutical ingredient. Hydrophilic liquids include, but are not limited to, water, juice, syrup, milk or an alcoholic beverage optionally containing an excipient. In some embodiments, the controlled pressure is between 50 and 100 bars, and the controlled temperature is between 30°C and 70°C. The spraying of the API solution into the aqueous cyclodextrin solution leads to the formation of API droplets that disperse in the aqueous cyclodextrin solution, and produces water-soluble cyclodextrin-encapsulated API concentrates. The aqueous cyclodextrin solution may comprise stabilizers, thickening agents and surfactants to enhance the stability of the API compounds in the solution.

The disclosed method produces pharmaceutical grade highly bioavailable soluble or drinkable solutions or suspensions comprising ultrafine cyclodextrin-encapsulated active pharmaceutical ingredients. The cyclodextrin-encapsulated API solutions and suspensions are ready for consumption without any further preparation, and may be diluted in water, hydrophilic liquids, brewed or fermented alcoholic and non-alcoholic beverages, juices, or any other drinkable liquid.

The active pharmaceutical ingredient processed according to the disclosed methods is a cannabinoid that may be present in any form.

The APIs may be in form of crude plant extracts, distillates, refined distillates, twice-refined distillates, three time-refined distillates or isolates. Plant extracts may contain plant material, such as lipids and waxes, chlorophyll, and terpenes, such as myrcene, geraniol, limonene, terpineol, pinene, menthol, thymol, carvacrol, camphor, and sesquiterpenes. Distillates may be prepared by mixing the extracts with alcohol and filtering the mixture to remove plant materials, followed by heating to remove the alcohol. For further refinement, the distillates may be heated to undergo short path distillation, and the process may be repeated several times to obtain twice-refined distillates, three time-refined distillates or isolates with a higher degree of purity. In alternative embodiments, the APIs may be in crystalline form.

Suitable cannabinoids and cannabinoid precursors include, but are not limited to, cannabigerolic acid (CBGA), cannabigerovaric acid (CBGVA, tetrahydrocannabinolic acid (THCA), cannabichromene acid (CBCA), cannabidiolic acid (CBDA), tetrahydrocannabivarinic acid (THCVA), cannabichromevarinic acid (CBCVA), cannabidivarinic acid (CBDVA), (-)-trans-Δ9-tetrahydrocannabinol (Δ9-THC), (-)-trans-Δ9-tetrahydrocannabipherol (Δ9-THCP),cannabigerol (CBG), cannabichromene (CBC), cannabicyclol (CBL), cannabidiol (CBD), cannabinodiol (CBND), cannabinol (CBN), analogs thereof, or any mixture thereof.

In some embodiments, the methods disclosed herein provide for cyclodextrin acetylation to increase the Lewis acid: Lewis base interactions of cyclodextrin with carbon dioxide and significantly increase their solubility. In other embodiments, the methods disclosed herein provide for the use of actylated cyclodextrins to increase API solubility in carbon dioxide, and hydrophilic cyclodextrins to form ultrafine cyclodextrin-encapsulated API inhalable powder. In other embodiments, the methods disclosed herein provide for the use of hydrophilic cyclodextrins to disperse API droplets and produce water-soluble API concentrates.

Suitable cyclodextrins include, but are not limited to, α-cyclodextrin, β-cyclodextrin and γ-cyclodextrin. Acetylated forms of cyclodextrin include, but are not limited to, α-cyclodextrin exadeacetate (AACD), β-cyclodextrin heneicosaacetate (ABCD), and γ-cyclodextrin octadeacetate (AGCD), respectively. Suitable hydrophilic cyclodextrines include, but are not limited to, hydrophilic α-cyclodextrin, hydrophilic β-cyclodextrin, hydrophilic γ-cyclodextrin and any mixture thereof.

The API extracts, distillates, refined distillates, twice-refined distillates, three time-refined distillates or high quality isolates may be combined with acetylated and/or hydrophilic cyclodextrins in API: cyclodextrin molar ratios ranging from 1:0.5 to 1:10. In some examples, the API: cyclodextrin molar ratio is 1:0.5, 1:0.75, 1:1, 1:1.5, 1:2, 1:2.5, 1:3, 1:3.5, 1:4, 1:4.5, 1:5, 1:5.5, 1:6, 1:6.5, 1:7, 1:7.5, 1:8, 1:8.5, 1:9, 1:9.5, or 1:10.

The API and the cyclodextrins may be mixed for a period of time that is defined by the type and form of the API used, the type of cyclodextrin used, temperature and pressure conditions, and the force used for mixing. In some embodiments, the preset pressure is in a range between 2,500 psi and 6,500 psi, and the preset temperature is in a range between 37°C and 55°C. Following pressurization, the API solution is depressurized at supersonic speed to induce particle formation, by releasing the API solution through a nozzle for short bursts. The diameter of the nozzle is in a range from 1 µm to 10 µm. In some embodiments, the diameter of the nozzle is 1 µm, 2 µm, 3 µm, 4 µm, 5 µm, 6 µm, or 7 µm. De-pressurization is best achieved by releasing the supercritical solution through the nozzle in short bursts such as, for example, 0.1, 0.2, 0.3, 0.4, 0.5, 0.6. 0.7, 0.8, 0.9 or 1 second bursts.

The supercritical, subcritical, high-pressure gas or liquid carbon dioxide may comprise an excipient or dispersing agent. In some embodiments, the disclosed methods may further comprise (vi) converting carbon dioxide into gas; (vii) filtering and pressuring carbon dioxide gas to achieve supercritical, subcritical, high-pressure gas or liquid status; and (viii) recirculating carbon dioxide in the reaction chamber for the next batch processing.

The cannabinoid fine nanoparticles produced by the methods provided herein have an average particle size between about 100 nm and about 40 µm and a size distribution within about 1% and about 50% of the average particle size.

The methods provided herein present numerous advantages. In particular, the disclosed methods significantly decrease API particle size, do not require the use of toxic organic solvents, and quickly and efficiently produce highly pure, ultrafine API-cyclodextrin inclusion complexes in form of nanoparticles, dry powder, solutions and suspensions, which are suitable for pulmonary and/or oral delivery. The cyclodextrin-encapsulated APIs produced by the disclosed methods are 99.9% pure, have 200% increased bioavailability compared to non- cyclodextrin-encapsulated active pharmaceutical ingredient formulations, and have excellent stability at room temperature for extended periods of time, such as 16 months, 24 months, 3 years, 4 years and 5 years.

### Apparatuses for Producing Pharmaceutical Grade, Pure, Ultrafine Cyclodextrin-Encapsulated APIs

Diagrams of exemplary apparatuses for performing the disclosed methods are shown in Figures 6, 7 and 8. However, any apparatus, system or equipment known in the art may be used to perform the methods provided herein.

In the diagram shown in Figure 6, canister 1 contains a 99% pure fluid, such as CO₂. The inlet valve 2 opens and controls the flow of the solvent fluid to the inlet accessing the HPLC pump 3. The outlet valve 4 opens and controls the flow of high-pressure solvent to the extraction vessel 8. The pressure gauge 5, which is integrated as part of the HPLC pump, indicates the pressure of the solvent in the inlet line and the extraction vessel 8. The temperature gauge 6 indicates the internal temperature of the extraction vessel 8. The heating bands 7 regulate the internal level of heat in the extraction vessel 8. The extraction vessel 8 contains the API with or without acetylated cyclodextrin to be dissolved in CO₂. Once the API solution is formed, the spray valve 9 depressurizes the API solution in the extraction vessel by releasing the solution through a spray nozzle 11 into the precipitation chamber 10, where the end product is collected. The pressure reaction valve or vent 12 reduces pressure in the precipitation chamber 10, and leads to spontaneous formation of ultrafine API nanoparticles or dry powder, which can then be collected and sorted according to their size.

In the diagram shown in Figure 7, an API and one or more acetylated cyclodextrins are inserted through a feeding valve into a heated pressurized vessel 1. Supercritical, subcritical, high-pressure gas or liquid carbon dioxide is then released from a CO2 tank through a feeding valve 5, chilled in a cooling chamber 3, and pumped with a pump 4 through an inlet valve 6 into the heated pressurized vessel 1 to dissolve the API and the acetylated cyclodextrins into a cyclodextrin-encapsulated API solution. The solution is then passed through a transfer valve 8, depressurized through a nozzle 9 with short bursts, collected into a powder collection vessel 2, and sorted by particle size through a final product outlet 10.

In the diagram shown in Figure 8, one or more hydrophilic cyclodextrins are fed through a feeding valve 22 into a heated pressurized vessel 12 and dissolved in a hydrophilic liquid at a pressure controlled through a pressure control valve 21 and at controlled temperature to form a hydrophilic cyclodextrin aqueous solution. An API is inserted through a feeding valve 17 into a heated pressurized vessel 11. Supercritical, subcritical, high-pressure gas or liquid carbon dioxide is then released from a CO2 tank through a feeding valve 15, chilled in a cooling chamber 13, and pumped with a pump 14 through an inlet valve 16 into the heated pressurized vessel 11 to dissolve the API. The API solution is then passed through a transfer valve 18, and depressurized through a nozzle 19 with short bursts into the heated pressurized vessel 12, where the droplets of API solution are dispersed into the aqueous cyclodextrin solution. The water-soluble hydrophilic API concentrates thus formed are collected through a final product outlet 20.

### Pharmaceutical Grade Ultrafine Cyclodextrin-Encapsulated APIs

Additionally provided herein are stable edible, inhalable, soluble or drinkable pharmaceutical grade cyclodextrin-encapsulated active pharmaceutical ingredients that are produced by the disclosed methods, as defined in the claims. The stable edible, inhalable, soluble or drinkable pharmaceutical grade cyclodextrin-encapsulated active pharmaceutical ingredients have 99.9% purity and 200% increased bioavailability compared to non-cyclodextrin-encapsulated active pharmaceutical ingredient formulations. The active pharmaceutical ingredient is a cannabinoid.

The disclosed edible, inhalable, soluble or drinkable pharmaceutical grade cyclodextrin-encapsulated active pharmaceutical ingredients may be formulated as compositions for oral, pulmonary, enteral, parenteral, intravenous, topical, mucosal, and submucosal administration, as prescribed, non-prescribed and retail provision of medical and pharmaceutical products, for the treatment, prevention, and alleviation of diseases, disorders, ailments and complaints, including, but not limited to, Alzheimer's Disease, epilepsy, mild and chronic pain, chemotherapy-induced peripheral neuropathy, insomnia, opioid and drug addiction, addiction sparing, inflammatory lung disease, anxiety disorders, PTSD, panic attacks, phobias, allergies, respiratory difficulty impairments and diseases, including coronaviruses, asthma and COPD, and menieres disease.

In some embodiments, the pharmaceutical grade cyclodextrin-encapsulated active pharmaceutical ingredient is in form of inhalable nanoparticles having an average particle size between 100 nm and 40 µm and a size distribution within 1% and 50% of the average particle size.

In some embodiments, the pharmaceutical grade cyclodextrin-encapsulated active pharmaceutical ingredient is in form of inhalable ultrafine dry powder having an average particle size between 100 nm and 5 µm.

In some embodiments, the pharmaceutical grade cyclodextrin-encapsulated active pharmaceutical ingredient is in form of a drinkable or soluble solution or suspension.

Because of their stability, the disclosed edible, inhalable, soluble or drinkable pharmaceutical grade cyclodextrin-encapsulated active pharmaceutical ingredients may be easily manufactured, mixed with other comestible ingredients or preparations, consumed or distributed without any risk of resuspension or separation.

### EXAMPLES

### Example 1: Cannabinoid Extracts, Distillates and Isolates

Cannabinoid precursors cannabigerolic acid (CBGA) and cannabigerovaric acid (CBGVA) were obtained by extraction from *Cannabis* plants or commercially purchased. The cannabinoids tetrahydrocannabinolic acid (THCA), cannabinolic acid (CBDA), cannabichromene acid (CBCA), (-)-trans-Δ9-tetrahydrocannabinolic acid (Δ9-THCA), tetrahydrocannabivarinic acid (THCVA), cannabichromevarinic acid (CBCVA) and cannabidivarinic acid (CBDVA) were extracted from *Cannabis sativa* plants by organic solvent extraction, steam or supercritical fluid extraction. Neutral forms of cannabinoids, tetrahydrocannabinol (THC), cannabidiol (CBD), (-)-trans-Δ9-tetrahydrocannabinol (Δ9-THC), (-)-trans-Δ9-tetrahydrocannabipherol (Δ9-THCP),cannabigerol (CBG), cannabichromene (CBC), cannabicyclol (CBL), cannabidiol (CBD), cannabinodiol (CBND), and cannabinol (CBN), were obtained by decarboxylation of their corresponding acidic forms by heating, drying, or combustion. For decarboxylation by heating cannabinoid extracts were heated at 95°C for about 20 minutes until melted, and then cooled in a freezer for about 15 minutes.

The cannabinoid extracts were subject to molecular distillation, and the distillates were refined by removing terpenes, organic material and chlorophyll by thin layer chromatography (THLC), high performance liquid chromatography (HPLC), liquid chromatography-mass spectrometry and/or gas chromatography-flame ionization detector (GC-FID) analysis.

The cannabinoid liquid oil distillates obtained as described above were used as such. Alternatively, the refined cannabinoid liquid oil distillates were refined once more to obtain twice-distilled cannabinoids. Triple-distilled cannabinoid isolates with high purity were obtained by refining the twice-distilled cannabinoids a third time.

### Example 2: Preliminary Testing

Fine nanoparticles were produced as disclosed herein. The system was optimized to minimize the effect of humidity, by washing with CO₂ prior to cannabinoid addition, and the pressure release process was optimized to 0.5 seconds with a 25 second re-pressurization cycle to prevent the nozzle from freezing and ensure uniformity and reproducibility.

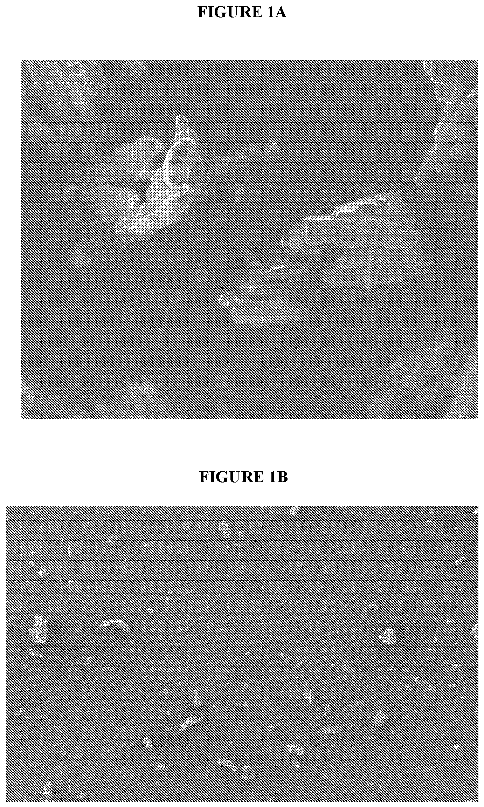

A cannabinoid in form of extract, distillate or isolate was added to a 10 ml high pressure reactor chamber and liquid CO₂ was pumped into the reactor chamber at a pressure of 1000 psi. The reactor was heated to 40°C and the pressure rose to a range from about 1500 psi to about 1700 psi. Temperature was kept at 40°C or was increased to 50°C. Pressure was then increased in 1000 psi increments from about 2500 psi to about 6500 psi using a syringe pump. A temperature of 40°C and a pressure of 3500 psi were selected for preliminary testing. The resultant solution was released through a 5µm nozzle for 0.5 second bursts. Figure 1A shows a CBD isolate prior to processing. The CBD isolate has crystalline morphology and a large amount of agglomeration between large particles. Figure 1B shows a CBD distillate after processing at a pressure of 3500 psi and a temperature of 40°C. The resulting distillate particles showed a more spherical amorphous morphology and had a particle size between 100 nm and 40 µm.

### Example 3: Complexation with Cyclodextrins

To increase cannabinoid solubility in water, the cannabinoid extracts, distillates and isolates produced as described in Example 1 were combined with α-cyclodextrin or β-cyclodextrin in cannabinoid: cyclodextrin molar ratios ranging from 1:0.5 to 1:10 and added to a 10 ml reactor chamber. Supercritical CO₂ was pumped into the reaction chamber at a pressure of 1,000 psi, the reactor chamber was heated to 40°C and the pressure was elevated to 3,500 psi. The resultant solution was released through a 5µm nozzle for 0.5 second bursts. Cyclodextrin was found to be insoluble under the process conditions.

To increase solubility in supercritical fluids, α-cyclodextrin and β-cyclodextrin were acetylated by substituting one or more hydroxyl groups with one or more acetyl groups to increase the Lewis acid: Lewis base interactions in supercritical fluid. 2.0 g of α-cyclodextrin, β-cyclodextrin or γ-cyclodextrin were acetylated in 10 ml acetic anhydride in a 100 ml round bottom flask. 0.05 g of iodine was added to the mixture and the flask was stirred in the dark for 2 hours. The reaction was quenched with 50 ml of water, and 1% (w/w) aqueous sodium thiosulfate was added dropwise until the solution turned clear. The reaction was stirred for 1 hour, and the resulting solution was extracted with 4 portions of 40 ml of dichloromethane (DCM). The organic fractions were combined and washed twice with 50 ml water and dried over sodium sulfate prior to solvent removal. The final products were dried in vacuum to yield α-cyclodextrin exadeacetate (AACD), β-cyclodextrin heneicosaacetate (ABCD), or γ-cyclodextrin octadeacetate (AGCD), respectively.

The acetylated cyclodextrins were then complexed with the cannabinoid extracts, distillates and isolates in cannabinoid:cyclodextrin molar ratios ranging from 1:0.5 to 1:10 and added to a 10 ml reactor chamber. Supercritical CO₂ was pumped into the reaction chamber at a pressure of 1,000 psi, the reactor chamber was heated to 40°C and the pressure was elevated to 3,500 psi. The resultant solution was released through a 5µm nozzle for 0.5 second bursts.

The results showed that AACD, ABCD and AGCD solubility in supercritical CO₂ increased to 1.1 and 1.3 wt. % respectively, under experimental conditions. Furthermore, cannabinoid complexation with acetylated cyclodextrins prevented re-suspension of cannabinoids and impurities thereof, such as terpenes and waxes, during processing.

### Example 4: Preparation of Cannabinoid Ultrafine Nanoparticles

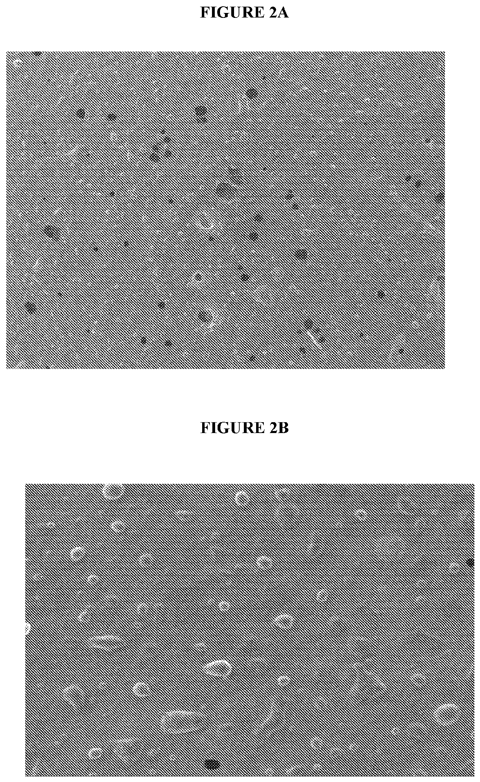

Cannabinoid complexes with acetylated cyclodextrins were prepared as described in Example 3 in cannabinoid: cyclodextrin molar ratios ranging from 1:0.5 to 1:10 and each added to a 10 ml reactor chamber. The cannabinoid-cyclodextrin complexes were dissolved in supercritical fluid at a pressure of 3500 psi and a temperature of 40°C. The solution was depressurized through a 5-micron nozzle into a 19-liter expansion chamber with tubular exhaust to ensure maximum recovery of particulates. Figures 2A and 2B show a 32X magnification and a 200X magnification of CBD distillate particles complexed with α-cyclodextrin in a cannabinoid: cyclodextrin molar ratio of 1:2.5 w/w (250 mg of CBD complexed with 100 mg α-cyclodextrin), respectively. The produced CBD nanoparticles showed spherical morphology with a particle size between 100 nm and 40 µm, and addition of acetylated cyclodextrins produced a fine powder that did not resuspend after processing, suggesting integration of the CBD compound into the AACD ring as shown in Figures 2A and 2B.

### Example 5: Bioavailability of Cannabinoid Ultrafine Nanoparticles

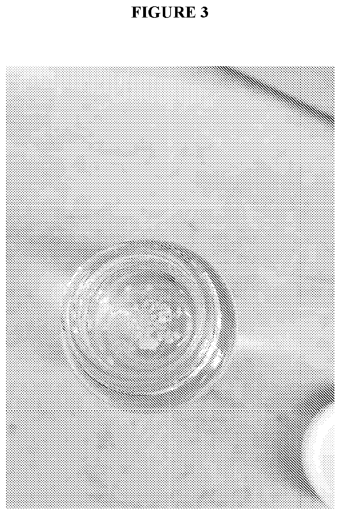

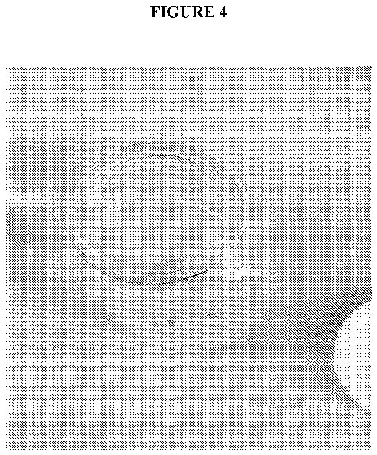

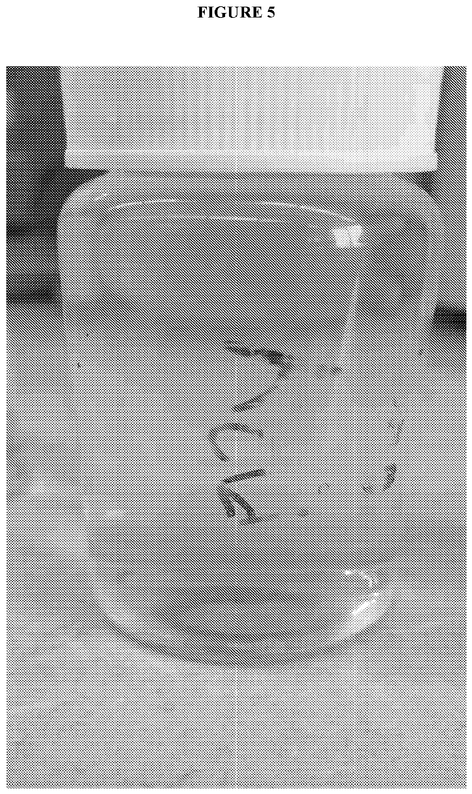

Bioavailability of the cannabinoid ultrafine nanoparticles obtained as described in Example 4 was assessed by visually evaluating the solubility of the fine nanoparticles in simulated stomach conditions. 0.5 g of NaCl was added to a 0.155 M solution of HCl in water to replicate stomach acidic conditions. 10 mg of the fine nanoparticles, 10 mg of the isolates in crystalline form, and 10 mg of the distillates were each placed in vials containing 10 ml of the acidic solution and incubated for 10 hours at 37°C. At the end of the 10-hour period, only minimal solubility of the preparations was observed. Additional 10 ml of the acidic solution were added, and the mixtures were incubated for 10 more hours at 37°C. At the end of the 20-hour period, the cannabinoid nanoparticles dissolved in the acidic solution. In contrast, isolates in crystalline form and distillates showed complete insolubility (Figures 3-5).

### Example 6: Relative Bioavailability Test of Cannabinoid Ultrafine Nanoparticles

Relative bioavailability tests of the cannabinoid ultrafine nanoparticles obtained as described in Example 4 (test samples) as compared to cannabinoid isolates in water (control samples) were performed using a high performance liquid chromatography (HPLC) separator equipped with a UV detector to determine the concentration of CBD in each sample. Control samples were prepared by filtering 1 ml of each sample through a 0.45µm filter into a 2 ml HPLC vial and 1 ml of methanol (MeOH) was added to each sample vial. HPLC mobile phase was comprised of 65% acetonitrile and 35% water. A flow rate of 1 ml per minute led to the elution of CBD after approximately 4.5 minutes.

A percentage area was measured after 32 hours elapsed time, which represents the amount of CBD in each sample relative to the background signal created by the MeOH in each sample. It was found that the percentage area of the test samples was 4.1163% of the total sample as compared to a percentage area of 0.7706% of the total sample for the control samples.

These results indicate that the disclosed purified cannabinoid fine nanoparticles enhance the solubility of CBD when compared to control cannabinoid isolates in water. Significant increases in solubility (in this instance up to 6 fold) indicate a potential for dramatic improvements in the bioavailability of the disclosed formulations. Significant increases in bioavailability can dramatically improve therapeutic effects.

### Example 7: Preparation of Water-Soluble Cyclodextrin-Encapsulated API Nanoparticles

To increase API solubility in water, the cannabinoid distillates as described in Example 1 were combined with various cyclodextrins, and the resultant mixtures were placed in a high-pressure reactor. Liquefied CO₂ was pumped into the reactor until the reactor pressure reached 5,000psi. The mixtures were agitated for 30 minutes in the reactor to create cyclodextrin-encapsulated cannabinoids. The mixtures were then sprayed into a cyclone to allow CO₂ to evaporate and obtain cyclodextrin-encapsulated cannabinoid dry powder. The recovered CO₂ was stored in a buffer tank for future use. Table 1 below shows the percentage cannabinoid amount in each sample. Table 1 also shows that the average percentage cannabinoid amount in the cyclodextrin-encapsulated cannabinoid nanoparticles was 10 times higher than the average cannabinoid amount in standard non cyclodextrin-encapsulated cannabinoid nanoparticles.

**TABLE 1**

| Component | % | Average % | STD |
|---|---|---|---|
| Δ9-THC | 8.540 | **10.94** | 1.42 |
| Δ9-THC | 11.860 | | |
| Δ9-THC | 12.474 | | |
| Δ9-THC | 11.813 | | |
| Δ9-THC | 10.481 | | |
| Δ9-THC | 10.497 | | |

### Example 8: Dissolution Profile of Cyclodextrin-Encapsulated API Powders

Dissolution profiles were determined by dissolving the samples obtained from Example 7. Commercial THC oil (Reign Drops, THC 30mg/ml) was used as standard control. Each sample containing equivalent amount of cannabinoids (40mg) were dissolved in 200ml of distilled water. The temperature was kept constant at 50°C.

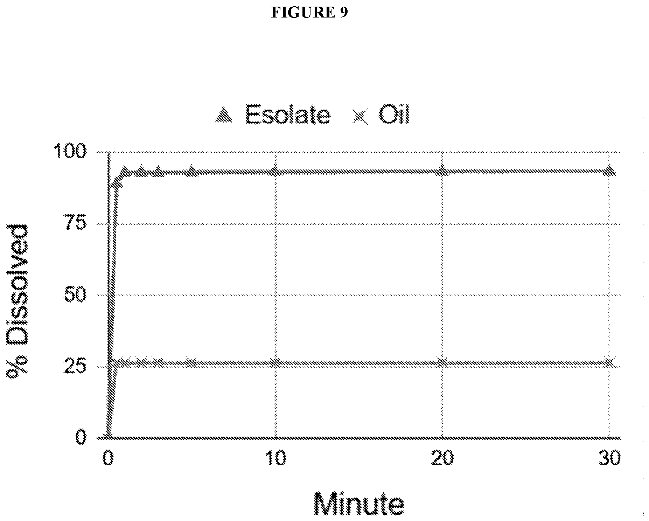

At time intervals 0.5, 1, 2, 3, 5, 10, 20, and 30 minutes, 2ml of each sample solution were withdrawn from the medium and immediately filtered through 0.45µm syringe filters. The filtered solutions were then analyzed by HPLC at 220nm wavelength using 0.085% phosphoric acid in methanol and 0.085% phosphoric acid in water as mobile phases. The results, summarized in Table 2 below and depicted in Figure 9, show that over 90% of cyclodextrin-encapsulated API dry powder is dissolved in water. In contrast, only 26% of standard control non cyclodextrin-encapsulated cannabinoid dry powder is dissolved in water. These results confirmed that cyclodextrin-encapsulated APIs have superior bioavailability and effectiveness when compared to non cyclodextrin-encapsulated APIs.

**TABLE 2**

| Cyclodextrin-Encapsulated API Powder | | | | |
|---|---|---|---|---|
| Minutes | API Concentration (w/v%) | | Average (w/v%) | % dissolved |
| 0.5 | 17.1875 | 18.64501 | 17.916255 | 89.25 |
| 1 | 18.66619 | 18.66063 | 18.66341 | 92.97 |
| 2 | 18.70305 | 18.63969 | 18.67137 | 93.01 |
| 3 | 18.62934 | 18.66595 | 18.647645 | 92.89 |
| 5 | 18.69115 | 18.66815 | 18.67965 | 93.05 |
| 10 | 18.68348 | 18.71033 | 18.696905 | 93.14 |
| 20 | 18.73447 | 18.70816 | 18.721315 | 93.26 |
| 30 | 18.72417 | 18.74888 | 18.736525 | 93.33 |

| Standard Control THC Oil | | | | |
|---|---|---|---|---|
| Min | API Concentration (w/v%) | | Average (w/v%) | % dissolved |
| 0.5 | 5.49102 | 5.48626 | 5.48864 | 26.39 |
| 1 | 5.4935 | 5.4934 | 5.49345 | 26.41 |
| 2 | 5.48777 | 5.49362 | 5.490695 | 26.40 |
| 3 | 5.49263 | 5.5034 | 5.498015 | 26.43 |
| 5 | 5.4905 | 5.49752 | 5.49401 | 26.41 |
| 10 | 5.49222 | 5.49453 | 5.493375 | 26.41 |
| 20 | 5.49193 | 5.50197 | 5.49695 | 26.43 |
| 30 | 5.49995 | 5.49953 | 5.49974 | 26.44 |

### Example 9: In Vivo Absorption Testing of Cyclodextrin-Encapsulated Cannabinoids

Baker's yeast (*Saccharomyces cerevisiae*) was used to measure speed of transport across membranes and evaluate uptake of cyclodextrin-encapsulated cannabinoids into living organisms as compared to non-encapsulated THC absorption over a two-hour period.

Yeast were inoculated into a sugar solution and allowed to acclimatize for 15 minutes at 35°C. Half of the yeast cultures were then treated with a solution containing non-encapsulated THC as control, and half of the yeast cultures were treated with a solution containing an equivalent amount of THC in form of cyclodextrin-encapsulated THC in an equivalent amount. Treatment was for two hours at 35°C with gentle agitation to facilitate gas exchange. At the end of treatment, the solution was removed by centrifugation and the yeast cells were washed with saline solution, lysed and subject to organic extraction. The organic cannabinoid solution was analyzed by HPLC. The results shown in Table 3 below demonstrate that cyclodextrin microencapsulation enhances THC transport across the yeast membrane and THC absorption by 200% relative to the transport of non-encapsulated THC. Overall, these results demonstrate that cyclodextrin microencapsulation improves cannabinoid absorption in eukaryotic systems such as humans, and can provide an enhanced recreational or medical experience in users.

**TABLE 3**

| Cyclodextrin-Encapsulated API Powder | | | | |
|---|---|---|---|---|
| Minutes | API Concentration (w/v%) | | Average (w/v%) | % Absorbed |
| 15 | 12.07262 | 10.32666 | 11.19964 | 55.79 |
| 30 | 11.51032 | 15.05445 | 13.282385 | 66.16 |
| 45 | 12.76902 | 11.44861 | 12.108815 | 60.32 |

| Standard Control THC Oil | | | | |
|---|---|---|---|---|
| Minutes | API Concentration (w/v%) | | Average (w/v%) | % Absorbed |
| 15 | 10.12045 | 5.90382 | 8.012135 | 38.52 |
| 30 | 5.52736 | 5.94221 | 5.734785 | 27.57 |
| 45 | 4.93078 | 3.77447 | 4.352625 | 20.93 |

It should be recognized that illustrated embodiments are only examples of the disclosed methods. The invention is defined by the following claims.

## Claims

1. A method of producing a stable edible, inhalable, soluble or drinkable pharmaceutical grade highly bioavailable and stable ultrafine cyclodextrin-encapsulated active pharmaceutical ingredient having 99.9% purity, wherein the active pharmaceutical ingredient is a cannabinoid, and wherein the method comprises in non-sequential order:
(a) dissolving the active pharmaceutical ingredient (API) in supercritical, subcritical, high-pressure gas or liquid carbon dioxide to form an API solution;
(b) adding one or more cyclodextrins to the API solution;
(c) pumping the carbon dioxide at a set pressure and a set temperature for a pre-determined period of time;
(d) depressurizing the API solution; and
(e) spraying the API solution, thereby producing a stable edible, inhalable, soluble or drinkable pharmaceutical grade highly bioavailable and stable ultrafine cyclodextrin-encapsulated active pharmaceutical ingredient.

2. The method of claim 1, wherein the pharmaceutical grade highly bioavailable fine cyclodextrin-encapsulated active pharmaceutical ingredient is in form of inhalable ultrafine nanoparticles having an average particle size between 100 nm and 40 µm and a size distribution within about 1% and about 50% of the average particle size, and wherein the method comprises:
(i) dissolving the API and one or more acetylated cyclodextrins in supercritical, subcritical, high-pressure gas or liquid carbon dioxide in a reaction chamber;
(ii) pumping the carbon dioxide at a set pressure and a set temperature for a pre-determined period of time to obtain an acetylated cyclodextrin-encapsulated API solution;
(iii) depressurizing the acetylated cyclodextrin-encapsulated API solution;
(iv) spraying the acetylated cyclodextrin-encapsulated API solution into a heated precipitator and through a nozzle to obtain inhalable ultrafine nanoparticles of acetylated cyclodextrin-encapsulated active pharmaceutical ingredient; and
(v) collecting and sorting the inhalable ultrafine nanoparticles of acetylated cyclodextrin-encapsulated active pharmaceutical ingredient by particle size.

3. The method of claim 1, wherein the pharmaceutical grade highly bioavailable and stable ultrafine cyclodextrin-encapsulated active pharmaceutical ingredient is in form of inhalable dry powder, and wherein the method comprises:
(i) pulverizing hydrophilic cyclodextrin into particles having an average particle size between 100 nm and 5 µm;
(ii) dissolving the API and one or more acetylated cyclodextrins in supercritical, subcritical, high-pressure gas or liquid carbon dioxide in the reaction chamber;
(iii) pumping the carbon dioxide at a set pressure and a set temperature for a pre-determined period of time to obtain an acetylated cyclodextrin-encapsulated API solution;
(iv) depressurizing the acetylated cyclodextrin-encapsulated API solution;
(v) adding hydrophilic cyclodextrin particles to the acetylated cyclodextrin-encapsulated API solution to create a hydrophilic cyclodextrin suspension-acetylated cyclodextrin-encapsulated API solution mixture;
(vi) spraying the mixture into a heated precipitator and through a nozzle to obtain inhalable ultrafine dry powder of a cyclodextrin-encapsulated active pharmaceutical ingredient; and
(vii) collecting and sorting the inhalable ultrafine dry powder of the cyclodextrin-encapsulated active pharmaceutical ingredient by particle size.

4. The method of claim 1, wherein the pharmaceutical grade highly bioavailable and stable ultrafine cyclodextrin-encapsulated active pharmaceutical ingredient is in form of a soluble or drinkable solution or suspension, and wherein the method comprises:
(i) dissolving hydrophilic cyclodextrin in a hydrophilic liquid at controlled pressure and temperature to form a hydrophilic cyclodextrin aqueous solution;
(ii) dissolving the API in supercritical, subcritical, high-pressure gas or liquid carbon dioxide in a reaction chamber;
(iii) pumping the carbon dioxide at a set pressure and a set temperature for a pre-determined period of time to obtain an API solution;
(iv) depressurizing the API solution; and
(v) spraying the API solution into the hydrophilic cyclodextrin aqueous solution and through a nozzle to obtain a drinkable solution or suspension of a hydrophilic cyclodextrin-encapsulated active pharmaceutical ingredient.

5. The method of any one of the preceding claims, wherein the supercritical, subcritical, high-pressure gas or liquid carbon dioxide comprises an excipient or dispersing agent, and wherein the method further comprises (vi) converting carbon dioxide into gas; (vii) filtering and pressuring carbon dioxide gas to achieve supercritical, subcritical, high-pressure gas or liquid status; and (viii) recirculating carbon dioxide in the reaction chamber.

6. The method of claim 1, wherein the cannabinoid comprises one or more of cannabigerolic acid (CBGA), cannabigerovaric acid (CBGVA, tetrahydrocannabinolic acid (THCA), cannabichromene acid (CBCA), cannabidiolic acid (CBDA), tetrahydrocannabivarinic acid (THCVA), cannabichromevarinic acid (CBCVA), cannabidivarinic acid (CBDVA), (-)-trans-Δ9-tetrahydrocannabinol (Δ9-THC), (-)-trans-Δ9-tetrahydrocannabipherol (Δ9-THCP), cannabigerol (CBG), cannabichromene (CBC), cannabicyclol (CBL), cannabidiol (CBD), cannabinodiol (CBND), cannabinol (CBN), or any mixture thereof; and wherein the one or more cannabinoids are in form of an extract, a distillate, a twice-refined distillate, a triple-refined distillate, or a partially purified isolate prior to processing.

7. The method of claim 2 or claim 3, wherein the one or more acetylated cyclodextrins comprise acetylated α-cyclodextrin, acetylated β-cyclodextrin, acetylated γ-cyclodextrin or any mixture thereof; and wherein the API and the one or more acetylated cyclodextrins are in an API: acetylated cyclodextrin molar ratio ranging from 1:0.5 to 1:10, or wherein the API: acetylated cyclodextrin molar ratio is 1:0.5, 1:0.75, 1:1, 1:1.5, 1:2, 1:2.5, 1:3, 1:3.5, 1:4, 1:4.5, 1:5, 1:5.5, 1:6, 1:6.5, 1:7, 1:7.5, 1:8, 1:8.5, 1:9, 1:9.5, or 1:10.

8. The method of claim 4, wherein the hydrophilic cyclodextrin comprises hydrophilic α-cyclodextrin, hydrophilic β-cyclodextrin, hydrophilic γ-cyclodextrin or any mixture thereof; and wherein the hydrophilic liquid is water, juice, syrup, milk or an alcoholic or non-alcoholic beverage optionally containing an excipient.

9. A stable edible, inhalable, soluble or drinkable pharmaceutical grade cyclodextrin-encapsulated active pharmaceutical ingredient having 99.9% purity, wherein the active pharmaceutical ingredient is a cannabinoid, wherein the pharmaceutical grade cyclodextrin-encapsulated active pharmaceutical ingredient is in form of inhalable nanoparticles having an average particle size between 100 nm and 40 µm and a size distribution within 1% and 50% of the average particle size, and wherein the nanoparticles are produced by the method of claim 2.

10. A stable edible, inhalable, soluble or drinkable pharmaceutical grade cyclodextrin-encapsulated active pharmaceutical ingredient having 99.9% purity, wherein the active pharmaceutical ingredient is a cannabinoid, wherein the pharmaceutical grade cyclodextrin-encapsulated active pharmaceutical ingredient is in form of inhalable ultrafine dry powder having an average particle size between 100 nm and 5 µm, and wherein the inhalable ultrafine dry powder is produced by the method of claim 3.

11. A stable edible, inhalable, soluble or drinkable pharmaceutical grade cyclodextrin-encapsulated active pharmaceutical ingredient having 99.9% purity, wherein the active pharmaceutical ingredient is a cannabinoid, wherein the pharmaceutical grade cyclodextrin-encapsulated active pharmaceutical ingredient is in form of a soluble or drinkable solution or suspension, and wherein the soluble or drinkable solution or suspension is produced by the method of claim 4.

12. The stable edible, inhalable, soluble or drinkable pharmaceutical grade cyclodextrin-encapsulated active pharmaceutical ingredient of claim 9 or claim 10, wherein the one or more acetylated cyclodextrins comprise acetylated α-cyclodextrin, acetylated β-cyclodextrin, acetylated γ-cyclodextrin or any mixture thereof; and wherein the API and the one or more acetylated cyclodextrins are in an API: acetylated cyclodextrin molar ratio ranging from 1:0.5 to 1:10.

13. The stable edible, inhalable, soluble or drinkable pharmaceutical grade cyclodextrin-encapsulated active pharmaceutical ingredient of any one of claims 9-12, wherein the supercritical, subcritical, high-pressure gas or liquid carbon dioxide comprises an excipient or dispersing agent, and wherein the method further comprises (vi) converting carbon dioxide into gas; (vii) filtering and pressuring carbon dioxide gas to achieve supercritical, subcritical, high-pressure gas or liquid status; and (viii) recirculating carbon dioxide in the reaction chamber.

## Patentansprüche

1. Verfahren zur Herstellung eines stabilen essbaren, inhalierbaren, löslichen oder trinkbaren, pharmazeutischen Wirkstoffs von pharmazeutischer Qualität, der hoch bioverfügbar und stabil, ultrafein und mit Cyclodextrin eingekapselt ist und eine Reinheit von 99,9 % aufweist, wobei der pharmazeutische Wirkstoff ein Cannabinoid ist und wobei das Verfahren in nicht-sequentieller Reihenfolge umfasst:
(a) Auflösen des pharmazeutischen Wirkstoffs (API) in überkritischem, unterkritischem, unter hohem Druck stehendem gasförmigem oder flüssigem Kohlendioxid, um eine API-Lösung zu bilden;
(b) Zugeben eines oder mehrerer Cyclodextrine zu der API-Lösung;
(c) Pumpen des Kohlendioxids bei einem festgelegten Druck und einer festgelegten Temperatur über einen vorbestimmten Zeitraum;
(d) Druckentlasten der API-Lösung; und
(e) Sprühen der API-Lösung, wodurch ein stabiler essbarer, inhalierbarer, löslicher oder trinkbarer, pharmazeutischer Wirkstoff von pharmazeutischer Qualität hergestellt wird, der hoch bioverfügbar und stabil, ultrafein und mit Cyclodextrin eingekapselt ist.

2. Verfahren nach Anspruch 1, wobei der pharmazeutische Wirkstoff von pharmazeutischer Qualität, der hoch bioverfügbar, fein und mit Cyclodextrin eingekapselt ist, in Form inhalierbarer ultrafeiner Nanopartikel mit einer durchschnittlichen Partikelgröße zwischen 100 nm und 40 µm und einer Größenverteilung innerhalb von etwa 1 % und etwa 50 % der durchschnittlichen Partikelgröße vorliegt, und wobei das Verfahren umfasst:
(i) Auflösen des API und eines oder mehrerer acetylierter Cyclodextrine in überkritischem, unterkritischem, unter hohem Druck stehendem gasförmigem oder flüssigem Kohlendioxid in einer Reaktionskammer;
(ii) Pumpen des Kohlendioxids bei einem festgelegten Druck und einer festgelegten Temperatur über einen vorbestimmten Zeitraum, um eine mit acetyliertem Cyclodextrin eingekapselte API-Lösung zu erhalten;
(iii) Druckentlasten der mit acetyliertem Cyclodextrin eingekapselten API-Lösung;
(iv) Sprühen der mit acetyliertem Cyclodextrin eingekapselten API-Lösung in einen beheizten Fällapparat und durch eine Düse, um inhalierbare ultrafeine Nanopartikel eines mit acetyliertem Cyclodextrin eingekapselten pharmazeutischen Wirkstoffs zu erhalten; und
(v) Sammeln uvnd Sortieren der inhalierbaren ultrafeinen Nanopartikel des mit acetyliertem Cyclodextrin eingekapselten pharmazeutischen Wirkstoffs nach Partikelgröße.

3. Verfahren nach Anspruch 1, wobei der pharmazeutische Wirkstoff von pharmazeutischer Qualität, der hoch bioverfügbar und stabil, ultrafein und mit Cyclodextrin eingekapselt ist, in Form eines inhalierbaren Trockenpulvers vorliegt, und wobei das Verfahren umfasst:
(i) Zerkleinern eines hydrophilen Cyclodextrins zu Partikeln mit einer durchschnittlichen Partikelgröße zwischen 100 nm und 5 µm;
(ii) Auflösen des API und eines oder mehrerer acetylierter Cyclodextrine in überkritischem, unterkritischem, unter hohem Druck stehendem gasförmigem oder flüssigem Kohlendioxid in der Reaktionskammer;
(iii) Pumpen des Kohlendioxids bei einem festgelegten Druck und einer festgelegten Temperatur über einen vorbestimmten Zeitraum, um eine mit acetyliertem Cyclodextrin eingekapselte API-Lösung zu erhalten;
(iv) Druckentlasten der mit acetyliertem Cyclodextrin eingekapselten API-Lösung;
(v) Zugeben von hydrophilen Cyclodextrinpartikeln zu der mit acetyliertem Cyclodextrin eingekapselten API-Lösung, um eine Mischung aus einer Suspension des hydrophilen Cyclodextrins und der mit acetyliertem Cyclodextrin eingekapselten API-Lösung zu erzeugen;
(vi) Sprühen der Mischung in einen beheizten Fällapparat und durch eine Düse, um ein inhalierbares ultrafeines Trockenpulver eines mit Cyclodextrin eingekapselten pharmazeutischen Wirkstoffs zu erhalten; und
(vii) Sammeln und Sortieren des inhalierbaren ultrafeinen Trockenpulvers des mit Cyclodextrin eingekapselten pharmazeutischen Wirkstoffs nach Partikelgröße.

4. Verfahren nach Anspruch 1, wobei der pharmazeutische Wirkstoff von pharmazeutischer Qualität, der hoch bioverfügbar und stabil, ultrafein und mit Cyclodextrin eingekapselt ist, in Form einer löslichen oder trinkbaren Lösung oder Suspension vorliegt, und wobei das Verfahren umfasst:
(i) Auflösen von hydrophilem Cyclodextrin in einer hydrophilen Flüssigkeit bei kontrolliertem Druck und kontrollierter Temperatur, um eine wässrige Lösung des hydrophilen Cyclodextrins zu bilden;
(ii) Auflösen des API in überkritischem, unterkritischem, unter hohem Druck stehendem gasförmigem oder flüssigem Kohlendioxid in einer Reaktionskammer;
(iii) Pumpen des Kohlendioxids bei einem festgelegten Druck und einer festgelegten Temperatur über einen vorbestimmten Zeitraum, um eine API-Lösung zu erhalten;
(iv) Druckentlasten der API-Lösung; und
(v) Sprühen der API-Lösung in die wässrige Lösung hydrophilen Cyclodextrins und durch eine Düse, um eine trinkbare Lösung oder Suspension eines mit hydrophilem Cyclodextrin eingekapselten pharmazeutischen Wirkstoffs zu erhalten.

5. Verfahren nach einem der vorhergehenden Ansprüche, wobei das überkritische, unterkritische, unter hohem Druck stehende gasförmige oder flüssige Kohlendioxid einen Hilfsstoff oder ein Dispergiermittel aufweist, und wobei das Verfahren ferner umfasst (vi) Umwandeln von Kohlendioxid in Gas; (vii) Filtern und Druckbeaufschlagen des Kohlendioxidgases, um einen überkritischen, unterkritischen, unter hohem Druck stehenden gasförmigen oder flüssigen Zustand zu erreichen; und (viii) Rezirkulieren des Kohlendioxids in der Reaktionskammer.

6. Verfahren nach Anspruch 1, wobei das Cannabinoid eines oder mehrere von Cannabigerolsäure (CBGA), Cannabigerovarsäure (CBGVA), Tetrahydrocannabinolsäure (THCA), Cannabichromensäure (CBCA), Cannabidiolsäure (CBDA), Tetrahydrocannabivarinsäure (THCVA), Cannabichromevarinsäure (CBCVA), Cannabidivarinsäure (CBDVA), (-)-Trans-Δ9-tetrahydrocannabinol (Δ9-THC), (-)-Trans-Δ9-tetrahydrocannabipherol (Δ9-THCP), Cannabigerol (CBG), Cannabichromen (CBC), Cannabicyclol (CBL), Cannabidiol (CBD), Cannabinodiol (CBND), Cannabinol (CBN) oder eine beliebige Mischung davon umfasst; und wobei das eine oder die mehreren Cannabinoide vor der Verarbeitung in Form eines Extrakts, eines Destillats, eines zweifach raffinierten Destillats, eines dreifach raffinierten Destillats oder eines teilweise gereinigten Isolats vorliegen.

7. Verfahren nach Anspruch 2 oder Anspruch 3, wobei das eine oder die mehreren acetylierten Cyclodextrine acetyliertes α-Cyclodextrin, acetyliertes β-Cyclodextrin, acetyliertes γ-Cyclodextrin oder eine beliebige Mischung davon umfassen; und wobei das API und das eine oder die mehreren acetylierten Cyclodextrine in einem molaren API:acetyliertes-Cyclodextrin-Verhältnis im Bereich von 1:0,5 bis 1:10 vorliegen oder wobei das molare API:acetyliertes-Cyclodextrin-Verhältnis 1:0,5, 1:0,75, 1:1, 1:1,5, 1:2, 1:2,5, 1:3, 1:3,5, 1:4, 1:4,5, 1:5, 1:5,5, 1:6, 1:6,5, 1:7, 1:7,5, 1:8, 1:8,5, 1:9, 1:9,5 oder 1:10 beträgt.

8. Verfahren nach Anspruch 4, wobei das hydrophile Cyclodextrin hydrophiles α-Cyclodextrin, hydrophiles β-Cyclodextrin, hydrophiles γ-Cyclodextrin oder eine beliebige Mischung davon umfasst; und wobei die hydrophile Flüssigkeit Wasser, Saft, Sirup, Milch oder ein alkoholisches oder nicht-alkoholisches Getränk ist, das gegebenenfalls einen Hilfsstoff enthält.

9. Stabiler essbarer, inhalierbarer, löslicher oder trinkbarer, pharmazeutischer Wirkstoff von pharmazeutischer Qualität, der mit Cyclodextrin eingekapselt ist und eine Reinheit von 99,9 % aufweist, wobei der pharmazeutische Wirkstoff ein Cannabinoid ist, wobei der pharmazeutische Wirkstoff von pharmazeutischer Qualität, der mit Cyclodextrin eingekapselt ist, in Form inhalierbarer Nanopartikel mit einer durchschnittlichen Partikelgröße zwischen 100 nm und 40 µm und einer Größenverteilung innerhalb von 1 % und 50 % der durchschnittlichen Partikelgröße vorliegt, und wobei die Nanopartikel durch das Verfahren nach Anspruch 2 hergestellt sind.

10. Stabiler essbarer, inhalierbarer, löslicher oder trinkbarer, pharmazeutischer Wirkstoff von pharmazeutischer Qualität, der mit Cyclodextrin eingekapselt ist und eine Reinheit von 99,9 % aufweist, wobei der pharmazeutische Wirkstoff ein Cannabinoid ist, wobei der pharmazeutische Wirkstoff von pharmazeutischer Qualität, der mit Cyclodextrin eingekapselt ist, in Form eines inhalierbaren ultrafeinen Trockenpulvers mit einer durchschnittlichen Partikelgröße zwischen 100 nm und 5 µm vorliegt, und wobei das inhalierbare ultrafeine Trockenpulver durch das Verfahren nach Anspruch 3 hergestellt ist.

11. Stabiler essbarer, inhalierbarer, löslicher oder trinkbarer, pharmazeutischer Wirkstoff von pharmazeutischer Qualität, der mit Cyclodextrin eingekapselt ist und eine Reinheit von 99,9 % aufweist, wobei der pharmazeutische Wirkstoff ein Cannabinoid ist, wobei der pharmazeutische Wirkstoff von pharmazeutischer Qualität, der mit Cyclodextrin eingekapselt ist, in Form einer löslichen oder trinkbaren Lösung oder Suspension vorliegt, und wobei die lösliche oder trinkbare Lösung oder Suspension durch das Verfahren nach Anspruch 4 hergestellt ist.

12. Stabiler essbarer, inhalierbarer, löslicher oder trinkbarer, pharmazeutischer Wirkstoff von pharmazeutischer Qualität, der mit Cyclodextrin eingekapselt ist, nach Anspruch 9 oder Anspruch 10, wobei das eine oder die mehreren acetylierten Cyclodextrine acetyliertes α-Cyclodextrin, acetyliertes β-Cyclodextrin, acetyliertes γ-Cyclodextrin oder eine beliebige Mischung davon umfassen; und wobei das API und das eine oder die mehreren acetylierten Cyclodextrine in einem molaren API:acetyliertes-Cyclodextrin-Verhältnis im Bereich von 1:0,5 bis 1:10 vorliegen.

13. Stabiler essbarer, inhalierbarer, löslicher oder trinkbarer, pharmazeutischer Wirkstoff von pharmazeutischer Qualität, der mit Cyclodextrin eingekapselt ist, nach einem der Ansprüche 9-12, wobei das überkritische, unterkritische, unter hohem Druck stehende gasförmige oder flüssige Kohlendioxid einen Hilfsstoff oder ein Dispergiermittel aufweist, und wobei das Verfahren ferner umfasst (vi) Umwandeln von Kohlendioxid in Gas; (vii) Filtern und Druckbeaufschlagen des Kohlendioxidgases, um einen überkritischen, unterkritischen, unter hohem Druck stehenden gasförmigen oder flüssigen Zustand zu erreichen; und (viii) Rezirkulieren des Kohlendioxids in der Reaktionskammer.

## Revendications

1. Procédé de production d'un principe actif pharmaceutique encapsulé dans de la cyclodextrine, ultrafin, stable, hautement biodisponible et de qualité pharmaceutique, comestible, inhalable, soluble ou buvable stable, ayant une pureté de 99,9 %, dans lequel le principe actif pharmaceutique est un cannabinoïde, et dans lequel le procédé comprend, dans un ordre non séquentiel :
(a) la dissolution du principe actif pharmaceutique (API) dans du gaz supercritique, sous-critique, à haute pression ou du dioxyde de carbone liquide pour former une solution d'API ;
(b) l'ajout d'une ou plusieurs cyclodextrines à la solution d'API ;
(c) le pompage du dioxyde de carbone à une pression et à une température définies pendant une durée prédéterminée ;
(d) la dépressurisation de la solution d'API ; et
(e) la pulvérisation de la solution d'API, produisant ainsi un principe actif pharmaceutique encapsulé dans de la cyclodextrine, ultrafin, stable, hautement biodisponible et de qualité pharmaceutique, comestible, inhalable, soluble ou buvable stable.

2. Procédé selon la revendicaton 1, dans lequel le principe actif pharmaceutique encapsulé dans de la cyclodextrine, fin, hautement biodisponible et de qualité pharmaceutique, se présente sous la forme de nanoparticules ultrafines inhalables ayant une taille de particule moyenne comprise entre 100 nm et 40 µm et une distribution de taille comprise entre environ 1 % et environ 50 % de la taille de particule moyenne, et dans lequel le procédé comprend :
(i) la dissolution de l'API et d'une ou plusieurs cyclodextrines acétylées dans du gaz supercritique, sous-critique, à haute pression ou du dioxyde de carbone liquide dans une chambre de réaction ;
(ii) le pompage du dioxyde de carbone à une pression et à une température définies pendant une durée prédéterminée pour obtenir une solution d'API encapsulé dans de la cyclodextrine acétylée ;
(iii) la dépressurisation de la solution d'API encapsulé dans de la cyclodextrine acétylée ;
(iv) la pulvérisation de la solution d'API encapsulé dans de la cyclodextrine acétylée dans un précipitateur chauffé et à travers une buse pour obtenir des nanoparticules ultrafines inhalables de principe actif pharmaceutique encapsulé dans de la cyclodextrine acétylée ; et
(v) la collecte et le tri des nanoparticules ultrafines inhalables de principe actif pharmaceutique encapsulé dans de la cyclodextrine acétylée en fonction de la taille de particule.

3. Procédé selon la revendicaton 1, dans lequel le principe actif pharmaceutique encapsulé dans de la cyclodextrine ultrafin, stable, hautement biodisponible et de qualité pharmaceutique, se présente sous la forme d'une poudre sèche inhalable, et dans lequel le procédé comprend :
(i) la pulvérisation de cyclodextrine hydrophile en particules ayant une taille de particule moyenne comprise entre 100 nm et 5 µm ;
(ii) la dissolution de l'API et d'une ou plusieurs cyclodextrines acétylées dans du gaz supercritique, sous-critique, à haute pression ou du dioxyde de carbone liquide dans la chambre de réaction ;
(iii) le pompage du dioxyde de carbone à une pression et à une température définies pendant une durée prédéterminée pour obtenir une solution d'API encapsulé dans de la cyclodextrine acétylée ;
(iv) la dépressurisation de la solution d'API encapsulé dans de la cyclodextrine acétylée ;
(v) l'ajout de particules de cyclodextrine hydrophile à la solution d'API encapsulé dans de la cyclodextrine acétylée pour créer un mélange de suspension de cyclodextrine hydrophile et de solution d'API encapsulé dans de la cyclodextrine acétylée ;
(vi) la pulvérisation du mélange dans un précipitateur chauffé et à travers une buse pour obtenir une poudre sèche ultrafine inhalable d'un principe actif pharmaceutique encapsulé dans de la cyclodextrine ; et
(vi) la collecte et le tri de la poudre sèche ultrafine inhalable du principe actif pharmaceutique encapsulé dans la cyclodextrine en fonction de la taille de particule.

4. Procédé selon la revendicaton 1, dans lequel le principe actif pharmaceutique encapsulé dans de la cyclodextrine ultrafin, stable, hautement biodisponible et de qualité pharmaceutique se présente sous la forme d'une solution ou d'une suspension soluble ou buvable, et dans lequel le procédé comprend :
(i) la dissolution de cyclodextrine hydrophile dans un liquide hydrophile à une pression et à une température contrôlées pour former une solution aqueuse de cyclodextrine hydrophile ;
(ii) la dissolution de l'API dans du gaz supercritique, sous-critique, à haute pression ou du dioxyde de carbone liquide dans une chambre de réaction ;
(iii) le pompage du dioxyde de carbone à une pression et à une température définies pendant une durée prédéterminée pour obtenir une solution d'API ;
(iv) la dépressurisation de la solution d'API ; et
(v) la pulvérisation de la solution d'API dans la solution aqueuse de cyclodextrine hydrophile et à travers une buse pour obtenir une solution ou une suspension buvable d'un principe actif pharmaceutique encapsulé dans de la cyclodextrine hydrophile.

5. Procédé selon l'une quelconque des revendications précédentes, dans lequel le gaz supercritique, sous-critique, à haute pression ou le dioxyde de carbone liquide comprend un excipient ou un agent dispersant, et dans lequel le procédé comprend en outre (vi) la conversion du dioxyde de carbone en gaz ; (vii) la filtration et la mise sous pression du dioxyde de carbone gazeux pour atteindre l'état de gaz supercritique, sous-critique, à haute pression ou de liquide ; et (viii) la recirculation du dioxyde de carbone dans la chambre de réaction.

6. Procédé selon la revendicaton 1, dans lequel le cannabinoïde comprend un ou plusieurs éléments parmi l'acide cannabigérolique (CBGA), l'acide cannabigérovarique (CBGVA), l'acide tétrahydrocannabinolique (THCA), l'acide cannabichroménique (CBCA), l'acide cannabidiolique (CBDA), l'acide tétrahydrocannabivarinique (THCVA), l'acide cannabichromévarinique (CBCVA), l'acide cannabidivarinique (CBDVA), le (-)-trans-Δ9-tétrahydrocannabinol (Δ9-THC), le (-)-trans-Δ9-tétrahydrocanna-biphérol (Δ9-THCP), le cannabigérol (CBG), le cannabichromène (CBC), le cannabicyclol (CBL), le cannabidiol (CBD), le cannabinodiol (CBND), le cannabinol (CBN), ou un mélange quelconque de ceux-ci ; et dans lequel les un ou plusieurs canna-binoïdes sont sous la forme d'un extrait, d'un distillat, d'un distillat doublement raffiné, d'un distillat triplement raffiné ou d'un isolat partiellement purifié avant le traitement.

7. Procédé selon la revendicaton 2 ou la revendicaton 3, dans lequel les une ou plusieurs cyclodextrines acétylées comprennent l'α-cyclodextrine acétylée, la B-cyclodextrine acétylée, la γ-cyclodextrine acétylée ou un mélange quelconque de celles-ci ; et dans lequel l'API et les une ou plusieurs cyclodextrines acétylées sont présents selon un rapport molaire API:cyclodextrine acétylée allant de 1:0,5 à 1:10, ou dans lequel le rapport molaire API:cyclodextrine acétylée est de 1:0,5, 1:0,75, 1:1, 1:1,5, 1:2, 1:2,5, 1:3, 1:3,5, 1:4, 1:4,5, 1:5, 1:5,5, 1:6, 1:6,5, 1:7, 1:7,5, 1:8, 1:8,5, 1:9, 1:9,5 ou 1:10.

8. Procédé selon la revendicaton 4, dans lequel la cyclodextrine hydrophile comprend l'α-cyclodextrine hydrophile, la β-cyclodextrine hydrophile, la γ-cyclodextrine hydrophile ou un mélange quelconque de celles-ci ; et dans lequel le liquide hydrophile est de l'eau, du jus, du sirop, du lait ou une boisson alcoolisée ou non alcoolisée contenant éventuellement un excipient.

9. Principe actif pharmaceutique encapsulé dans de la cyclodextrine, de qualité pharmaceutique, stable, comestible, inhalable, soluble ou buvable, présentant une pureté de 99,9 %, dans lequel le principe actif pharmaceutique est un cannabinoïde, dans lequel le principe actif pharmaceutique encapsulé dans de la cyclodextrine de qualité pharmaceutique se présente sous la forme de nanoparticules inhalables ayant une taille de particule moyenne comprise entre 100 nm et 40 µm et une distribution de taille comprise entre 1 % et 50 % de la taille de particule moyenne, et dans lequel les nanoparticules sont produites par le procédé selon la revendicaton 2.

10. Principe actif pharmaceutique encapsulé dans de la cyclodextrine de qualité pharmaceutique, stable, comestible, inhalable, soluble ou buvable, présentant une pureté de 99,9 %, dans lequel le principe actif pharmaceutique est un cannabinoïde, dans lequel le principe actif pharmaceutique encapsulé dans de la cyclodextrine de qualité pharmaceutique se présente sous la forme d'une poudre sèche ultrafine inhalable ayant une taille de particule moyenne comprise entre 100 nm et 5 µm, et dans lequel la poudre sèche ultrafine inhalable est produite par le procédé selon la revendicaton 3.

11. Principe actif pharmaceutique encapsulé dans de la cyclodextrine de qualité pharmaceutique, stable, comestible, inhalable, soluble ou buvable, présentant une pureté de 99,9 %, dans lequel le principe actif pharmaceutique est un cannabinoïde, dans lequel le principe actif pharmaceutique encapsulé dans de la cyclodextrine de qualité pharmaceutique se présente sous la forme d'une solution ou d'une suspension soluble ou buvable, et dans lequel la solution ou la suspension soluble ou buvable est produite par le procédé selon la revendicaton 4.

12. Principe actif pharmaceutique encapsulé dans de la cyclodextrine de qualité pharmaceutique, stable, comestible, inhalable, soluble ou buvable selon la revendicaton 9 ou la revendicaton 10, dans lequel les une ou plusieurs cyclodextrines acétylées comprennent l'α-cyclodextrine acétylée, la β-cyclodextrine acétylée, la γ-cyclodextrine acétylée ou un mélange quelconque de celles-ci ; et dans lequel l'API et les une ou plusieurs cyclodextrines acétylées sont présents selon un rapport molaire API:cyclodextrine acétylée allant de 1:0,5 à 1:10.

13. Principe actif pharmaceutique encapsulé dans de la cyclodextrine de qualité pharmaceutique, stable, comestible, inhalable, soluble ou buvable selon l'une quelconque des revendicatons 9 à 12, dans lequel le gaz supercritique, sous-critique, à haute pression ou le dioxyde de carbone liquide comprend un excipient ou un agent dispersant, et dans lequel le procédé comprend en outre (vi) la conversion de dioxyde de carbone en gaz ; (vii) la filtration et la mise sous pression de dioxyde de carbone gazeux pour atteindre l'état de gaz supercritique, sous-critique, à haute pression ou liquide ; et (viii) la recirculation du dioxyde de carbone dans la chambre de réaction.
